(19) Europäisches Patentamt
European Patent Office
Office européen des brevets

(11) Publication number: 0 272 909
A2

# (12) EUROPEAN PATENT APPLICATION

(21) Application number: **87311269.2**

(22) Date of filing: **22.12.87**

(51) Int. Cl.4: **C 07 C 153/05**

(30) Priority: **24.12.86 US 946337**

(43) Date of publication of application:
**29.06.88 Bulletin 88/26**

(84) Designated Contracting States:
**AT BE CH DE ES FR GB GR IT LI NL SE**

(71) Applicant: **ELI LILLY AND COMPANY**
**Lilly Corporate Center**
**Indianapolis Indiana 46285 (US)**

(72) Inventor: **Moder, Kenneth Philip**
**210 Wood Dale Street**
**West Lafayette Indiana 47906 (US)**

**Busch, Frank Robert**
**6 Pheasant Run Drive**
**Gales Ferry Connecticut 06335 (US)**

(74) Representative: **Tapping, Kenneth George et al**
**Erl Wood Manor**
**Windlesham Surrey, GU20 6PH (GB)**

## (54) Synthesis of aminothioacetamides.

(57) The present invention provides a novel process for preparing aminothioacetamides comprising reacting an aminonitrile with hydrogen sulfide in the presence of an alkali metal sulfide or hydrosulfide. The aminothioacetamides produced by the present novel process are useful as starting materials in the synthesis of pharmaceutically useful anti-ulcer agents.

EP 0 272 909 A2

## Description

### SYNTHESIS OF AMINOTHIOACETAMIDES

This invention provides a novel process for preparing aminothioacetamides.

Aminothioacetamides are useful as starting materials in the synthesis of pharmaceutical agents. For example, U.S. Patent No. 4,375,547 discloses N-alkyl-N′-([2-(aminoalkyl)-4-thiazolylmethyl]thioalkyl)guanidine, thiourea, and ethanediamine anti-ulcer agents derived from aminothioacetamides. Aminothioacetamides generally are prepared by reacting hydrogen sulfide with an aminoacetonitrile in the presence of an amine base such as ammonia, pyridine, or triethylamine. Vasil'eva et al., J. Org. Chem., (Russia), 6 (4), 882 (1970), illustrates this process by preparing dimethylaminothioacetamide in 56% non-isolated yield.

Unfortunately, the amine bases often interfere with isolation of the desired product If the amine base is removed by distillation, lower yields of the aminothioacetamide product result since the product co-distills with the amine base. If the amine base is not distilled, it also forms a salt when the reaction mixture is acidified, providing a highly impure final product.

According to the present invention there is provided a process for preparing aminothioacetamides of the formula

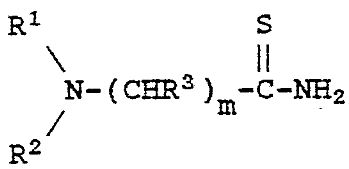

wherein:

$R^1$ and $R^2$ are independently H or $C_1$-$C_3$ alkyl, one of $R^1$ and $R^2$ can be benzyl or benzoyl, and when taken together with the nitrogen atom to which they are attached, represent piperidino, pyrrolidino, or morpholino; $R^3$ is H or $CH_3$; and m is 1, 2, or 3; comprising reacting an aminonitrile of the formula

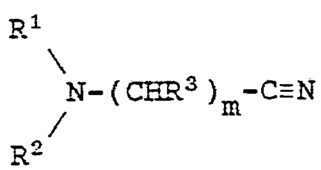

wherein $R^1$, $R^2$, $R^3$, and m are as defined above, with hydrogen sulfide in the presence of an alkali metal sulfide or hydrosulfide. Since the alkali metal sulfide or hydrosulfide is easily removed from the product aminothioacetamides, the aminothioacetamides can be isolated in higher yield and purity than if an amine base was used.

A preferred group of compounds which can be prepared by the present process are those wherein $R^1$ and $R^2$ are independently H or $C_1$-$C_3$ alkyl; $R^3$ is H; and m is 1.

The most preferred compound which can be prepared by the present process is dimethylaminothioacetamide.

The claimed process may be conducted by mixing an aminonitrile and an alkali metal sulfide or hydrosulfide in a neutral solvent. The aminonitrile starting materials employed are either commercially available, known in the literature, or can be prepared by methods known in the art. Suitable neutral solvents include non-cyclic amides such as N,N-dimethylformamide (DMF) and hexamethylphosphoramide (HMPA); cyclic amides such as 2-methylpyrrolidinone; alcohols such as methanol, ethanol, propanol and isopropanol; ethers such as tetrahydrofuran (THF), diethylether, and dioxane; and dimethylsulfoxide (DMSO). A preferred solvent is DMF. The concentration of aminonitrile in the solvent is not critical, but it is preferred to employ a sufficient amount of solvent to ensure that the product aminothioacetamide remains in solution throughout the reaction.

The alkali metal sulfide or hydrosulfide may be employed in either anhydrous or hydrated forms. Examples of acceptable alkali metal sulfides or hydrosulfides include sodium sulfide, sodium hydrosulfide, potassium sulfide, and potassium hydrosulfide. The sodium sulfide and sodium hydrosulfide alkali metal sulfides or hydrosulfides are preferred in the process of this invention.

Examples of acceptable forms of sodium sulfide include sodium sulfide anhydrous, sodium sulfide monohydrate, sodium sulfide pentahydrate, and sodium sulfide nonahydrate. The anhydrous and monohydrated forms are preferred. Examples of acceptable forms of sodium hydrosulfide include sodium hydrosulfide anhydrous, sodium hydrosulfide monohydrate, sodium hydrosulfide dihydrate, and sodium hydrosulfide trihydrate. A preferred sodium hydrosulfide form is the dihydrate.

The alkali metal sulfide or hydrosulfide generally is employed in a catalytic amount, although the amount is not critical. Typically the alkali metal sulfide or hydrosulfide is employed in a molar amount of about 0.1% to about 15.0% relative to the aminonitrile. The most preferred molar ratio employed is from about 0.5% to about 8.0%.

After mixing the aminonitrile, alkali metal sulfide or hydrosulfide, and solvent, hydrogen sulfide is added. The amount of hydrogen sulfide added will vary depending on the reaction temperature and pressure chosen. Desired reaction temperatures are within the range of about 10°C to about 80°C, with a preferred range being about 20°C to about 50°C. Desired reaction pressures are within the range of about atmospheric pressure (0 psig) to about 200 psig. A preferred reaction pressure range is from about 50 psig to about 130 psig.

The process of the present invention is substantially complete after about 5 hours to about 120 hours when conducted at temperatures in the range of about 10°C to about 80°C and pressures in the

range of about 0 psig to about 200 psig. The progress of the reaction can be followed, if desired, by standard high performance liquid chromatography (HPLC), or gas chromatography (GC), analytical techniques in order to determine when the reaction is substantially complete.

Once the process is substantially complete, the alkali metal sulfide or hydrosulfide is removed by filtration.

The alkylthioacetamide product may be isolated, if desired, by acidifying the reaction mixture to a pH of about 1.0 to about 5.0, for example by adding an anhydrous acid, such as hydrochloric acid or oxalic acid. The volatile organic constituents may be removed by evaporation under reduced pressure. A second solvent, such as tetrahydrofuran, anhydrous ethyl alcohol, or isopropanol, can be added and the mixture cooled to about 0°C. The product generally solidifies and can be isolated by standard isolation techniques, such as vacuum filtration or centrifugation, to afford the desired compound in good purity and high yield.

Alternatively, the product may be isolated by first removing all, or a portion of, the volatile organic constituents by evaporation under reduced pressure. The residue is then acidified per the above procedure, a second solvent added, the mixture cooled, and the product isolated by standard isolation techniques such as vacuum filtration or centrifugation.

If a concentrated solution of aminonitrile in the solvent is used to produce the product aminothioacetamide, the volatile organic constituents need not be removed prior to isolating the aminothioacetamide. The concentrated solution is merely acidified per the above procedure, a second solvent added, the mixture cooled, and the product isolated by standard isolation techniques such as vacuum filtration or centrifugation.

The following Examples illustrate specific aspects of the present invention. The Examples are not intended to limit the scope of the present process in any respect and should not be so construed.

Example 1

Dimethylaminothioacetamide Hydrochloride

To a pressure reactor charged with 51.0 g of 97.6% purity dimethylaminoacetonitrile (0.592 moles of dimethylaminoacetonitrile) dissolved in 400 ml of N,N-dimethylformamide (DMF) were added 2.60 g of sodium sulfide monohydrate. The reaction mixture was cooled to about -5.0°C, and the reactor charged with hydrogen sulfide until it had pressurized to about 120 psig and the reactor contents had warmed to about 18°C.

The reaction mixture was stirred for approximately 16 hours at a temperature and pressure of about 20°C and about 120 psig respectively, after which the pressure was slowly decreased to 60 psig over the next 68 hours. At that time the reaction, as evidenced by GC analysis, was substantially complete.

The reactor was vented to atmospheric pressure (0 psig) and the sodium sulfide removed by filtration. The reaction mixture was concentrated by vacuum distillation of approximately 75% of the DMF. Then 100 ml of DMF saturated with hydrochloric acid were added to the reaction mixture, lowering the pH to about 1.0.

Two hundred ml of tetrahydrofuran (THF) were added dropwise over a period of 5 minutes. The resulting mixture was cooled to -5°C, stirred, and then filtered. The filter cake was washed with 50 ml of THF and dried in a vacuum oven at 50°C for 24 hours to provide 77.0 g of dimethylaminothioacetamide hydrochloride (84.1% yield), m.p. 169.9°C-171.8°C. This product assayed 99.7% purity, when compared to reference standards using the HPLC assay discussed below.

The assay samples were dissolved in water. The column was eluted with an elution solvent comprised of 6.8 g of sodium acetate trihydrate, 3.0 ml of acetic acid, 1.0 ml of triethylamine, and 2.0 g of 1-heptanesulfonic acid sodium salt per liter of water. The column employed was a 15 cm × 4.6 mm ID Ultrasphere ODS. The detector had a wavelength of 254 nm, the column flow rate was 1.5 ml/min., and the injection volume was 10 μl.

Example 2

Dimethylaminothioacetamide Hydrochloride

To a pressure reactor charged with 78.33 g of dimethylaminoacetonitrile (0.931 mole) dissolved in 600 ml of DMF were added 0.81 g of sodium sulfide monohydrate. Hydrogen sulfide was added until the reactor was pressurized to about 80 psig. The contents of the reactor were heated to about 40°C, raising the reactor pressure to 120 psig.

The reaction mixture was stirred for approximately 28 hours at a temperature and pressure of about 40°C and about 120 psig respectively. The reaction was substantially complete, by GC analysis, so the reactor was vented to atmospheric pressure (0 psig) and the reaction mixture cooled to room temperature (25°C).

The sodium sulfide was removed by filtration. Then 110 ml of DMF saturated with hydrochloric acid were added, lowering the pH to about 5.0. The acidic mixture was heated to about 60°C and about 260 ml of DMF were removed using vacuum distillation.

The mixture was cooled to about 50°C and THF (400 ml) was added dropwise over a period of 10 minutes. The resulting mixture was cooled to -5°C, stirred, and then filtered. The filter cake was dried in a vacuum oven at 50°C for 24 hours to provide 114.9 g of dimethylaminothioacetamide hydrochloride (79.8% yield), m.p. 174.5°C-176.0°C. This product assayed 99.2% purity using the assay procedure discussed in Example 1.

Example 3

Dimethylaminothioacetamide Hydrochloride

To a pressure reactor charged with 50.0 g of 95.5% purity dimethylaminoacetonitrile (0.568 mole of dimethylaminoacetonitrile) dissolved in 200 ml of DMF were added 2.50 g of sodium hydrosulfide dihydrate. Hydrogen sulfide was added until the

reactor was pressurized to about 60 psig.

The reaction mixture was stirred for approximately 24 hours at a temperature and pressure of room temperature (25°C) and 60 psig respectively. The reaction was substantially complete, by GC analysis, so the reactor was vented to atmospheric pressure (0 psig).

The sodium hydrosulfide was removed by filtration. Anhydrous HCl gas was added until the solution pH was about 1.0. The acidic mixture was heated to about 60°C and approximately 80% of the DMF removed using vacuum distillation.

The mixture was cooled to about 30°C and anhydrous ethyl alcohol (200 ml) was added dropwise over a period of 5 minutes. The resulting mixture was heated to about 75°C for 15 minutes, then cooled down to -5°C, stirred, and then filtered. The filter cake was dried in a vacuum oven at 50°C for 24 hours to provide 78.7 g of dimethylaminothioacetamide hydrochloride (89.7% yield). This product assayed 99.4% purity using the assay procedure described in Example 1.

## Claims

1. A process for preparing an aminothioacetamide of the formula

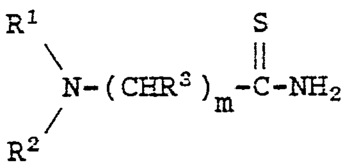

wherein:

$R^1$ and $R^2$ are independently H or $C_1$-$C_3$ alkyl, one of $R^1$ and $R^2$ can be benzyl or benzoyl, and when taken together with the nitrogen atom to which they are attached, represent piperidino, pyrrolidino, or morpholino; $R^3$ is H or $CH_3$; and m is 1, 2, or 3; comprising reacting an aminonitrile of the formula

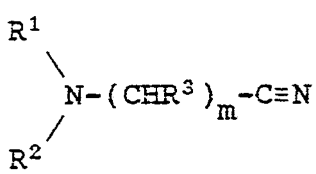

wherein $R^1$, $R^2$, $R^3$, and m are as defined above, with hydrogen sulfide in the presence of an alkali metal sulfide or hydrosulfide.

2. A process of claim 1 employing an aminonitrile wherein $R^1$ and $R^2$ are independently H or $C_1$-$C_3$ alkyl; $R^3$ is H; and m is 1.

3. A process of claim 1 wherein dimethylaminoacetonitrile is converted to dimethylaminothioacetamide.

4. A process of any one of claims 1-3 employing reaction temperatures from about 20°C to about 50°C.

5. A process of claim 4 employing pressures from about 50 psig to about 130 psig.

6. A process of any one of claims 1-5 employing either sodium sulfide anhydride or sodium sulfide monohydrate.

7. A process of claim 6 employing sodium sulfide monohydrate.

8. A process of any one of claims 1-5 employing sodium hydrosulfide dihydrate.

9. A process of claim 2 employing reaction pressures from about 50 psig to about 130 psig sodium sulfide monohydrate.

10. A process of claim 2 employing sodium hydrosulfide dihydrate.